# EUROPEAN PATENT SPECIFICATION

(11) **EP 3 723 855 B1**
(45) Date of publication and mention of the grant of the patent: **07.09.2022**
(21) Application number: 18842722.3
(22) Date of filing: 05.12.2018
(51) Int. Cl.: A61B 90/00, A61B 8/08, A61B 17/00, A61N 7/02, A61N 7/00, A61B 17/22

(54) **CONTROLLING DELIVERY OF THERAPEUTIC AGENT IN MICROBUBBLE-ENHANCED ULTRASOUND PROCEDURES**
STEUERUNG DER ABGABE VON THERAPEUTISCHEN MITTELN IN MIKROBLASENVERSTÄRKTEN ULTRASCHALLVERFAHREN
CONTRÔLE DE L'ADMINISTRATION D'AGENT THÉRAPEUTIQUE DANS DES PROCÉDURES ULTRASONORES RENFORCÉES PAR DES MICROBULLES

(30) Priority: 11.12.2017 US 201762597073 P; 11.12.2017 US 201762597076 P; 11.12.2017 US 201762597071 P
(43) Date of publication of application: 21.10.2020
(73) Proprietor: Insightec Ltd., 39120 Tirat-Carmel (IL)
(72) Inventor: ZADICARIO, Eyal, Tel Aviv-Yafo, OT (IL); LEVY, Yoav, Hinanit, OT (IL)
(74) Representative: HGF
(86) International application number: PCT/IB2018/001537
(87) International publication number: WO 2019/116095

(56) References cited:
- WO-A1-2011/025893
- WO-A2-2011/034892
- US-A1- 2008 269 668

## Description

### FIELD OF THE INVENTION

The present invention relates to systems for controlling microbubble characteristics (e.g., an agent type, a size, a concentration, a dose, an administration rate, pressure, or timing, and/or a location of an injection site) and delivery of therapeutic agents prior to and/or during procedures for increasing procedural efficiency.

### BACKGROUND

Focused ultrasound (i.e., acoustic waves having a frequency greater than about 20 kiloHertz) can be used to image or therapeutically treat internal body tissues within a patient. For example, ultrasound imaging systems produce images of body tissue by transmitting ultrasound waves to the body tissue and detecting and analyzing echoes reflected therefrom. In addition, ultrasound waves may be used in applications involving ablation of tumors, targeted drug delivery, disruption of the blood-brain barrier (BBB), lysing of clots, and other surgical procedures. During imaging and/or tumor ablation, a piezoceramic transducer is placed externally to the patient, but in close proximity to the tissue to be imaged and/or ablated (i.e., the target region). The transducer converts an electronic drive signal into mechanical vibrations, resulting in the emission of acoustic waves. The transducer may be geometrically shaped and positioned along with other such transducers so that the ultrasound energy they emit collectively forms a focused beam at a "focal zone" corresponding to (or within) the target region. Alternatively or additionally, a single transducer may be formed of a plurality of individually driven transducer elements whose phases can each be controlled independently. Such a "phased-array" transducer facilitates steering the focal zone to different locations by adjusting the relative phases among the transducers. As used herein, the term "element" means either an individual transducer in an array or an independently drivable portion of a single transducer. Magnetic resonance imaging (MRI) may be used to visualize the patient and target, and thereby to guide the ultrasound beam. MRI may also provide information about changes in the target resulting from the therapeutic effect and thus supports a feedback mechanism to control the therapy.

During a focused ultrasound procedure, small gas/liquid bubbles (or "microbubbles") may be generated and/or introduced into the target region. Depending upon the amplitude and frequency of the applied acoustic field, the microbubbles may oscillate or collapse (this mechanism is called "cavitation") and thereby cause various thermal effects in the target region and/or its surrounding region. For example, cavitation of microbubbles may enhance energy absorption at the ultrasound focal region such that the tissue therein may be heated faster and be ablated more efficiently than would occur in the absence of microbubbles. This effect and the response of the microbubbles to the ultrasound waves are referred to herein as the "microbubble response." If utilized in the central nervous system, microbubble cavitation may cause disruption of blood vessels, thereby inducing "opening" of the BBB for enhancing targeted drug delivery. Further, microbubbles may be employed for contrast enhanced ultrasound imaging, drug and gene delivery and/or metabolic gas delivery.

The mechanism of the microbubble response to application of the ultrasound waves involves a resonant property of the microbubbles - i.e., the microbubbles may oscillate at a resonance frequency in response to the applied acoustic field. The microbubble resonance frequency generally depends on the sizes of the microbubbles, the shell composition, the gas core composition, and the properties of the ambient medium in which they are present (e.g., a viscosity of the agent, a size of a vessel, etc.). Accordingly, one approach to controlling the microbubble response to the ultrasound waves is to control the sizes of the microbubbles. Microbubble preparations with various size distributions, shell compositions, and gas core compositions are available commercially. Accordingly, when the microbubble size optimal for an ultrasound procedure is determined, one of these preparations may be selected and administered to the patient. The microbubble size distribution may, however, be unstable - i.e., it may change with time; as a result, the microbubble response to the applied ultrasound may differ from what is expected, thereby reducing the clinical effect on the target tissue. In addition, even if the microbubble size distribution remains stable, the condition (e.g., the size or temperature) of the target tissue and/or non-target tissue may change during the ultrasound procedure, similarly detracting from the clinical effects of the microbubbles and potentially resulting in damage to non-target tissue.

Document WO 2011/034892 discloses a system wherein microbubbles flow through a grating, a sieve or similar so that the bubbles of undesired size are removed. Document WO 2011/025893 describes a system wherein the microbubbles of different sizes are separated by centrifugation. Document US 2008/269668 discloses a rotatable cradle used to separate microbubbles of different dimensions.

Accordingly, there is a need for improved microbubble-enhanced ultrasound procedures that reliably control the type, size, concentration, and administration of the microbubbles so as to optimize the performance of the procedure (e.g., optimize treatment effects on the target region) and avoid damage to non-target regions.

### SUMMARY

The present invention relates to an ultrasound system and an administration system for introducing the microbubbles into the patient's body during an ultrasound procedure. The ultrasound procedure may involve imaging the target region and/or non-target region (e.g., regions surrounding the target region and/or intervening regions located between the target region and the ultrasound system) or treating the target region (e.g., opening the BBB and/or ablating a target tumor). For ease of reference, the following description only refers to an ultrasound treatment procedure; it should be understood, however, that the same approaches generally apply as well to an ultrasound imaging procedure.

In various examples, prior to and/or during treatment, the optimal microbubble size for enhancing treatment effects is determined based on the characteristics of the target and/or non-target regions. As used herein, the terms "optimal" and "optimizing" generally involve determining and selecting the best microbubble size, shell composition, gas core composition, and/or size distribution practically discernible within the limitations of the utilized technology for imaging and/or treating the target region. The term "microbubble size" means the mean (or median or mode) microbubble radius in a distribution (e.g., a normal distribution) of microbubbles, a maximum microbubble radius, a normal size distribution around a specified mean or other size characteristic of the microbubbles.

Microbubbles having the determined optimal size may be manufactured using any suitable approach known to one of skill in the art or, alternatively, they may be acquired off-the-shelf. The size of the microbubbles to be administered may be verified using any suitable technique (e.g., laser light obscuration and scattering, electrozone sensing, optical microscopy, ultrasound or flow cytometry) prior to administration. If the measured size differs clinically significantly from the optimal size (e.g., by a factor of two or ten), a precautionary action may be taken. For example, the system may generate a warning signal to the user. Additionally or alternatively, the microbubbles may be centrifuged or otherwise fractionated to isolate the microbubbles having the desired size. Alternatively, and particularly in the case of an optimal maximum microbubble radius, the microbubbles may be filtered through a filter having a pore size corresponding to the determined size. The isolated/filtered microbubbles may then be verified again to ensure that the size thereof matches the optimal value; subsequently, the verified microbubbles may be administered to the patient to enhance ultrasound treatment. Accordingly, this approach allows substantially (e.g., > 50%, 90%, 95% or 99%) only the microbubbles having the desired characteristic to be administered to the patient.

In some embodiments, the administration profile (e.g., a dose, concentration, rate, timing or pressure) of the microbubbles is determined based on a characteristic (e.g., the diameter, size distribution, shell composition, or gas and/or liquid core composition) thereof prior to treatment. During the ultrasound procedure, the treatment effects (such as tissue-permeability enhancement, tissue disruption or ablation) of the target and/or non-target regions may be monitored and/or assessed using, for example, an imager, a contrast agent (e.g., a gadolinium-based contrast agent) and/or acoustic signals from the target and/or non-target regions. Based on the monitored treatment effects, the administration profile of the microbubbles and/or an ultrasound parameter (e.g., frequency, amplitude, phase, or activation time) may be adjusted in real time to optimize the treatment or achieve a desired target effect.

The ultrasound treatment procedure may be performed in combination with other therapeutic methods. For example, after the desired target effect on the target tissue is achieved, a therapeutic agent may be introduced for targeted drug delivery. Said therapeutic methods, however, are not a part of the invention.
Alternatively, a radiation device may be used therapeutically in conjunction with the ultrasound procedure. None of the said therapeutic methods constitute a part of the invention.

The administration system may include three independent channels fluidly coupled to three containers having the microbubbles, an MRI contrast agent, and a therapeutic agent, respectively. The three channels are also fluidly coupled to an introducing device (e.g., a catheter or a needle) for administering the microbubbles, contrast agent and/or therapeutic agent from their corresponding channels into the patient's body. The administration system may include an actuation mechanism (e.g., a syringe, a peristaltic pump, etc.) for dispensing the fluid from the container(s) to the patient. Operation of the actuation mechanism may be controlled by a controller. In addition, the controller may be in communication with the ultrasound controller to cause activation and deactivation of the transducer in order to optimize the treatment effect and/or destroy microbubbles that do not conform to the size criterion (e.g., the determined optimal microbubble size).

The administration system may include multiple fluidic channels coupled to the microbubble container; each channel may include a filter having a different pore size. During treatment, based on the condition (e.g., size, location, shape or temperature) of the target and/or non-target regions and/or a desired effect on acoustic energy delivery, the optimal microbubble size for achieving a treatment goal (e.g., maximizing the temperature at the target region and minimizing damage to non-target tissue) can be determined and dynamically updated. If the optimal size changes over the course of the procedure, the channel corresponding to the new optimal size can be activated, i.e., microbubbles are forced therethrough for administration to the patient. In this way, the size of the administered microbubbles can be controlled in real time. In addition, the control or administration system may adjust an administration profile (e.g., a concentration, an administering dose, rate, timing or pressure) of the microbubbles in real time during treatment based on the condition of the target/non-target region and/or the microbubble response.

Each of the microbubble channels may be coupled to one of a plurality of microbubble vials/containers each having microbubbles of a different size. During the ultrasound procedure, based on the condition of the target and/or non-target regions and/or the response of the microbubbles to the ultrasound, the channel corresponding to the updated optimal size is connected to a microbubble administration device (e.g., a catheter or a needle) for introducing the microbubbles into the patient's body. Again, the administration system may adjust the administration profile of the microbubbles, contrast agent, and/or therapeutic agent in the channels and/or in the introducing device based on real-time feedback of the target/non-target condition and/or microbubble response so as to optimize the treatment effects and/or minimize undesired damage to the non-target tissue.

The invention pertains to a system for microbubble-enhanced treatment of target tissue. In various embodiments, the system includes an administration device having the first storage container for storing microbubbles wherein the first storage container is connected to a plurality of channels, each having a manipulation means for changing the microbubble characteristic, wherein the manipulation means is configured to apply an acoustic field to the microbubbles; and a controller configured to receive a desired characteristic of the microbubbles for treating the target tissue; cause the microbubbles to be dispensed from the administration device; and compare a characteristic of the dispensed microbubbles to the desired characteristic so as to validate a match therebetween. The controller may be further configured to cause the dispensed microbubbles to be introduced to the target tissue upon validation of the match. Additionally or alternatively, the controller may be further configured to cause a precautionary action upon detecting a clinically significant deviation between the characteristic of the dispensed microbubbles from the desired characteristic.

The system may further include a measurement system for measuring the characteristic of the dispensed microbubbles. In one embodiment, the measurement system includes an acoustic system. The acoustic system is configured to apply multiple frequencies to the microbubbles for measuring the characteristic thereof. In addition, the acoustic system may be further configured to measure attenuation, scattering, backscattering, harmonic generation, and/or sub-harmonic generation from the microbubbles. In another embodiment, the measurement system is configured to perform measurements of a laser light obscuration and scattering, electrozone sensing, optical microscopy and/or flow cytometry. Additionally or alternatively, the measurement system may include one or more sensors for measuring an administration rate of the microbubbles.

The system further includes an ultrasound transducer for transmitting ultrasound waves to the microbubbles. The measurement system may then be configured to monitor a response of the microbubbles to the ultrasound waves. The controller may be further configured to adjust an ultrasound parameter (e.g., a frequency, a phase, and/or an amplitude) and/or the characteristic of the dispensed microbubbles based at least in part on the monitored response thereof. In some embodiments, the administration device further includes the second storage container for storing a therapeutic agent. The therapeutic agent may include Busulfan, Thiotepa, CCNU (lomustine), BCNU (carmustine), ACNU (nimustine), Temozolomide, Methotrexate, Topotecan, Cisplatin, Etoposide, Irinotecan /SN-38, Carboplatin, Doxorubicin, Vinblastine, Vincristine, Procarbazine, Paclitaxel, Fotemustine, Ifosfamide /4-Hydroxyifosfamide /aldoifosfamide, Bevacizumab, 5-Fluorouracil, Bleomycin, Hydroxyurea, Docetaxel, and/or Cytarabine (cytosine arabinoside, ara-C)/ara-U. The controller is further configured to cause the ultrasound transducer to transmit treatment ultrasound waves to the target tissue and generate an acoustic field therein; and cause administration of the therapeutic agent so as to produce an optimal treatment effect in the target tissue. In addition, the system may further include a measurement system (e.g., an imager and/or an acoustic-signal detector) for detecting signals indicating a condition of a treatment procedure. The condition of the treatment procedure may include a condition (e.g., a temperature, a size, a shape and/or a location) of the target tissue, a condition of the therapeutic agent, and/or a condition (e.g., a cavitation state) of the microbubbles.

The controller may be further configured to adjust the characteristic (e.g., an agent type, a size, a concentration, an administering rate, an administering dose, an administering pattern, an administering time and/or an administering pressure) of the administered microbubbles and/or the administered therapeutic agent based at least in part on the detected signals. In addition, the controller may be further configured to cause the ultrasound transducer to transmit ultrasound waves to the target tissue in the presence of the administered microbubbles. In some embodiments, the administration device further includes the third storage container for storing a contrast agent. In addition, the administration device further includes an actuation mechanism; an introducing device for delivering the microbubbles, therapeutic agent and/or contrast agent to the target tissue; and one or more channels for delivering the microbubbles, contrast agent and/or therapeutic agent from the corresponding storage container to the introducing device.

The first storage container is connected to multiple channels, each having a manipulation means for changing the microbubble characteristic. The manipulation means may include multiple filters having different exclusion sizes; the controller is then configured to select a channel having one of the filters corresponding to the desired microbubble characteristic; and cause the microbubbles to be administered only through the selected channel. Additionally or alternatively, the manipulation means may include applying an acoustic field to the microbubbles. The controller may then be further configured to tune a frequency band of the acoustic field so as to destroy the microbubbles having a size outside a desired size range. In addition, the system may further include multiple storage containers, each containing microbubbles having a different size characteristic; the controller is then configured to select one of the storage containers having therein microbubbles corresponding to the desired microbubble characteristic; and cause the microbubbles to be administered only from the selected storage container.

The system may further include a radiation device for transmitting a radiation dose to the target tissue. In addition, the controller may be further configured to dispense the microbubbles from the administration device based at least in part on a predetermined administration profile (e.g., a dose, a concentration, an administering rate, an administering timing and/or an administering pressure of the microbubbles). The administration profile may be determined based at least in part on the characteristic (e.g., a diameter, a size distribution, a shell composition, a gas composition and/or a liquid core composition) of the dispensed microbubbles. In some embodiments, the system further includes a preparation device for preparing the microbubbles based at least in part on the desired characteristic. In addition, the controller may be further configured to acquire a characteristic of the target tissue and determine the desired characteristic of the microbubbles based at least in part on the characteristic of the target tissue.

The invention relates to a system for microbubble-enhanced treatment of target tissue. The system includes an administration device having a storage container for storing microbubbles; and a controller configured to receive a desired characteristic of the microbubbles for treating the target tissue; and cause substantially only the microbubbles having the desired characteristic to be administered into the target tissue. In one implementation, the controller is further configured to acquire a characteristic of the target tissue and determine the desired characteristic of the microbubbles based at least in part on the characteristic of the target tissue.

The system may further include one or more fluidic channels coupled to the first storage container for delivering the microbubbles to the target tissue; and a manipulator associated with the fluidic channel(s) for changing therein the microbubble characteristic. The manipulator may include a filter having a determined size characteristic. Additionally or alternatively, the manipulator may include an ultrasound transducer for applying an acoustic field to the microbubbles in the fluidic channel(s). The transducer may be configured to apply an acoustic field having one or more frequency bands so as to destroy the microbubbles having a size outside a desired size range.

In some embodiments, the controller is further configured to administer the microbubbles based on a predetermined administration profile (e.g., a dose, a concentration, an administering rate, an administering timing or an administering pressure). The administration profile is determined based at least in part on the second characteristic (e.g., a diameter, a size distribution, a shell composition, and/or a gas and/or liquid core composition) of the microbubbles. In one embodiment, the system further includes a preparation device for preparing the microbubbles based at least in part on the desired characteristic, substantially all of the prepared microbubbles having the desired characteristic.

The invention relates to a system for microbubble-enhanced treatment of target tissue. The system includes an administration device having a storage container for storing microbubbles; and the system may also include a preparation device for preparing the microbubbles based at least in part on a desired characteristic (e.g., a maximum diameter, a mean diameter, a normal size distribution around a specified mean, a shell composition, or a gas and/or liquid core composition), substantially all of the prepared microbubbles having the desired characteristic. The preparation device may include a heater, a shaker and/or an ultrasound transducer.

The invention relates to a system for microbubble-enhanced treatment of target tissue. The system may include an ultrasound transducer having multiple transducer elements; an administration device having the first storage container for storing microbubbles and the second storage container for storing a therapeutic agent; a manipulator associated with the administration device for changing a characteristic of the microbubbles; a measurement system for detecting signals indicating a condition of a treatment procedure; and a controller. The controller may be configured to cause the ultrasound transducer to transmit ultrasound waves to the target tissue; cause the microbubbles and therapeutic agent to be dispensed from the administration device to the target tissue; cause the measurement system to detect the signals indicating the condition of the treatment procedure; and based at least in part on the detected signals, adjust (i) a parameter value (e.g., a frequency, a phase and/or an amplitude) associated with one or more transducer elements, (ii) the microbubble characteristic (e.g., a maximum diameter, a mean diameter, a normal size distribution around a specified mean, a shell composition, or a gas and/or liquid core composition), and/or (iii) an administration profile (e.g., a dose, a concentration, an administering rate, an administering timing, and/or an administering pressure) of the microbubbles and/or the therapeutic agent. In one implementation, the manipulator includes a filter having a determined size characteristic.

As used herein, the term "substantially" means ±10%, and in some embodiments, ±5%. Reference throughout this specification to "one example," "an example," "one embodiment," or "an embodiment" means that a particular feature, structure, or characteristic described in connection with the example is included in at least one example of the present technology. Thus, the occurrences of the phrases "in one example," "in an example," "one embodiment," or "an embodiment" in various places throughout this specification are not necessarily all referring to the same example. Furthermore, the particular features, structures, routines, steps, or characteristics may be combined in any suitable manner in one or more examples of the technology. The headings provided herein are for convenience only and are not intended to limit or interpret the scope or meaning of the claimed technology.

### BRIEF DESCRIPTION OF THE DRAWINGS

In the drawings, like reference characters generally refer to the same parts throughout the different views. Also, the drawings are not necessarily to scale, with an emphasis instead generally being placed upon illustrating the principles of the invention. In the following description, various embodiments of the present invention are described with reference to the following drawings, in which:
FIG. 1 illustrates a microbubble-enhanced focused ultrasound system in accordance with various embodiments;
FIG. 2 depicts an exemplary relationship between the microbubble size and the microbubble resonance frequency;
FIGS. 3A and 3B illustrate a time evolution of the microbubble size distribution
FIG. 4 is a flow chart illustrating an approach for selecting a desired size distribution of microbubbles and applying the selected microbubbles to enhance performance of an ultrasound procedure;
FIG. 5 is a flow chart illustrating an approach for treating a target utilizing a microbubble-enhanced ultrasound procedure in combination with other therapeutic methods
FIGS. 6A-6D depicts exemplary administration systems.

### DETAILED DESCRIPTION

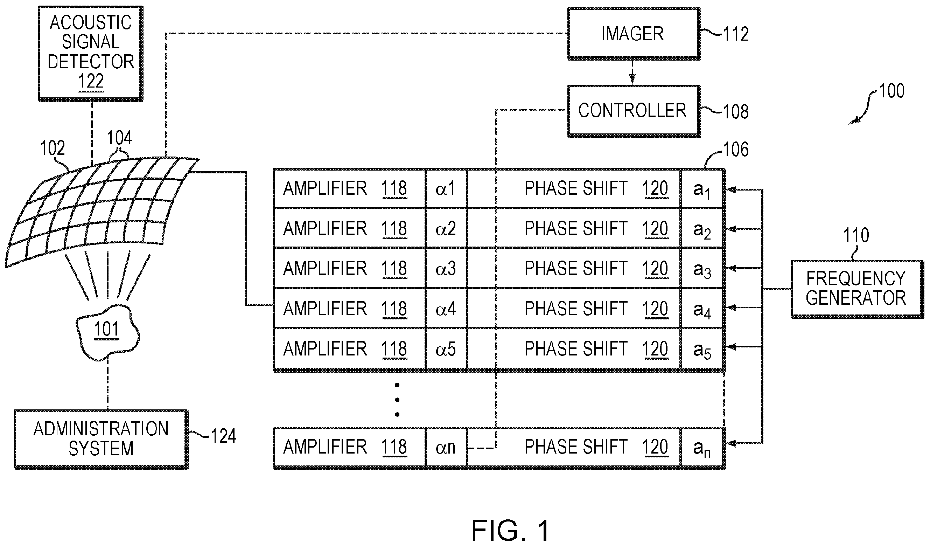

FIG. 1 illustrates an exemplary ultrasound system 100 for generating and delivering a focused acoustic energy beam to a target region 101 within a patient's body. The applied ultrasound waves may be reflected from the target region and/or non-target region, and an image of the target and/or non-target regions may be generated based on the reflected waves. In addition, microbubbles may be introduced to the target region 101 and/or non-target region to increase ultrasound reflections, thereby improving the contrast of the ultrasound image. In some embodiments, the applied ultrasound waves may ablate tissue in the target region 101 and/or induce microbubble oscillation and/or cavitation to improve treatment effects. The illustrated system 100 includes a phased array 102 of transducer elements 104, a beamformer 106 driving the phased array 102, a controller 108 in communication with the beamformer 106, and a frequency generator 110 providing an input electronic signal to the beamformer 106. In various embodiments, the system further includes an imager 112, such as a magnetic resonance imaging (MRI) device, a computer tomography (CT) device, a positron emission tomography (PET) device, a single-photon emission computed tomography (SPECT) device, or an ultrasonography device, for determining anatomical characteristics (e.g., the type, property, structure, thickness, density, etc.) of the tissue at the target region 101 and/or the non-target region.

The array 102 may have a curved (e.g., spherical or parabolic) shape suitable for placement on the surface of the patient's body, or may include one or more planar or otherwise shaped sections. Its dimensions may vary between millimeters and tens of centimeters. The transducer elements 104 of the array 102 may be piezoelectric ceramic elements, and may be mounted in silicone rubber or any other material suitable for damping the mechanical coupling between the elements 104. Piezo-composite materials, or generally any materials capable of converting electrical energy to acoustic energy, may also be used. To assure maximum power transfer to the transducer elements 104, the elements 104 may be configured for electrical resonance at 50 Ω, matching input connector impedance.

The transducer array 102 is coupled to the beamformer 106, which drives the individual transducer elements 104 so that they collectively produce a focused ultrasonic beam or field. For n transducer elements, the beamformer 106 may contain n driver circuits, each including or consisting of an amplifier 118 and a phase delay circuit 120; each drive circuit drives one of the transducer elements 104. The beamformer 106 receives a radio frequency (RF) input signal, typically in the range from 0.1 MHz to 10 MHz, from the frequency generator 110, which may, for example, be a Model DS345 generator available from Stanford Research Systems. The input signal may be split into n channels for the n amplifiers 118 and delay circuits 120 of the beamformer 106. In some embodiments, the frequency generator 110 is integrated with the beamformer 106. The radio frequency generator 110 and the beamformer 106 are configured to drive the individual transducer elements 104 of the transducer array 102 at the same frequency, but at different phases and/or different amplitudes.

The amplification or attenuation factors α₁-αₙ and the phase shifts a₁-aₙ imposed by the beamformer 106 serve to transmit and focus ultrasonic energy through the intervening tissue located between the transducer elements 104 and the target region onto the target region 101, and account for wave distortions induced in the intervening tissue. The amplification factors and phase shifts are computed using the controller 108, which may provide the computational functions through software, hardware, firmware, hardwiring, or any combination thereof. In various embodiments, the controller 108 utilizes a general-purpose or special-purpose digital data processor programmed with software in a conventional manner, and without undue experimentation, in order to determine an optimal value of an ultrasound parameter (e.g., a frequency, a phase shift and/or an amplification factor) associated with each element 104 so as to generate a desired focus or any other desired spatial field patterns. The optimal value of the ultrasound parameter may be refined experimentally before, after, and/or at one or more times during the ultrasound procedure based on, for example, the focus quality, the focus location relative to the target 101 and/or the microbubble response to the ultrasound sonications. The quality and location of the focus may be monitored using the imager 112, and the microbubble response may be detected using the transducer 102 and/or an acoustic-signal detector 122 (e.g., a hydrophone).

In certain embodiments, the optimal value of the ultrasound parameter is computationally estimated based on detailed information about the characteristics of the intervening tissue and their effects (e.g., reflection, refraction, and/or scattering) on propagation of acoustic energy. Such information may be obtained from the imager 112 and analyzed manually or computationally. Image acquisition may be three-dimensional or, alternatively, the imager 112 may provide a set of two-dimensional images suitable for reconstructing a three-dimensional image of the target and/or non-target regions. Image-manipulation functionality may be implemented in the imager 112, in the controller 108, or in a separate device.

In certain treatment scenarios, ultrasound waves propagating towards the target region 101 from different directions may encounter a highly variable anatomy, such as different thicknesses of tissue layers and different acoustic impedances; as a result, energy deposition at the target region 101 varies significantly and often nonmonotonically with frequency, and the optimum frequency for a particular patient is typically unpredictable. Accordingly, in some embodiments, the frequency of the ultrasound is optimized by sequentially sonicating the target region 101 with waves having different "test frequencies" within a test frequency range; for each tested frequency, a parameter (e.g., temperature, acoustic force, tissue displacement, etc.) indicative of energy deposition in the target region 101 is measured. The test range may span the entire range of frequencies suitable for ultrasound treatment (e.g., in various embodiments, 0.1 MHz to 10 MHz), but is typically a much smaller sub-range thereof within which the optimal frequency is expected. Such a sub-range may be determined, e.g., based on computational estimates of the optimal frequency, the results of simulations, or empirical data acquired for the same target in other patients. Further details about determining the optimal frequency for the ultrasound application are provided, for example, in U.S. Patent Publication No. 2016/0008633,

In some embodiments, optimizing the ultrasound frequency involves iteratively setting a test frequency, sonicating the target region 101 at the selected frequency, and quantitatively assessing the resulting focusing properties or energy deposition at the target region 101. This may be accomplished using, e.g., MRI thermometry to measure the temperature increase in the target region 101 resulting from the deposited energy, MR-ARFI to measure the tissue displacement resulting from the acoustic pressure at the target region 101, ultrasound detection to measure the intensity of the ultrasound reflected from the target region 101, or generally any experimental technique for measuring a parameter that correlates with energy deposition at the target region 101 in a known and predictable manner. Following frequency optimization, the phase and/or amplitude settings of the phased-array transducer 102 may be adjusted to optimize the focus for the selected frequency. Approaches to assessing focusing properties at the target region 101 and, based thereon, adjusting the ultrasound frequency, phase and/or amplitude settings of the phased-array transducer 102 are provided, for example, in an International Patent Application entitled "Adaptive, Closed-Loop Ultrasound Therapy" filed on even date herewith,

In various embodiments, microbubbles and/or other therapeutic agents are introduced intravenously or, in some cases, by injection proximate to the target region 101 using an administration system 124 for enhancing the ultrasound procedure on the target region. For example, the microbubbles may be introduced into the patient's brain in the form of liquid droplets that subsequently vaporize, or as gas-filled bubbles, or entrained with another suitable substance, such as a conventional ultrasound contrast agent. Because of their encapsulation of gas, the microbubbles may act as reflectors of the ultrasound. Reflections from the microbubbles are generally more intense than the reflections from the soft tissue of the body. Therefore, by combining the microbubbles with a contrast agent, the contrast level of an ultrasound image may be significantly increased.

In addition, the microbubbles may react to an applied oscillating acoustic pressure with volume pulsations. Depending on the amplitude of the ultrasound waves, microbubble oscillation will be related either linearly or nonlinearly to the applied acoustic pressure. For a relatively low acoustic pressure, the instantaneous radius of the microbubbles may oscillate linearly in relation to the amplitude of the applied acoustic pressure. Microbubble oscillation is governed by parameters such as the resonance frequency, damping coefficients, and shell properties. Thus, if the frequency of the applied acoustic waves is equal to the microbubble resonance frequency, the microbubbles may experience a large force and can collapse. This may result in undesired damage to the non-target tissue if the microbubbles are present therein.

To avoid this undesired effect, in various embodiments, the resonance frequency of the microbubbles is selected to be substantially different from (e.g., two times greater than) the selected optimal ultrasound frequency. In addition, the transducer elements 104 are activated to transmit waves having a low acoustic power (e.g., 5W) during treatment. In this way, because the ultrasound waves arriving the non-target region have a low acoustic intensity and their frequency is substantially different from the resonance frequency of the microbubbles, the microbubbles in the non-target region may be unresponsive (or have a limited response without cavitation) to the applied acoustic field. This ensures that no (or at least a very limited amount of) non-target tissue is damaged. In contrast, at the target region 101, where the ultrasound beams are focused, the acoustic intensity is substantially larger than that outside the target region and may be sufficient to cause the microbubbles to oscillate and/or collapse. This may enhance energy absorption at the target region 101 for tissue ablation and/or cause disruption of blood vessels for targeted drug delivery.

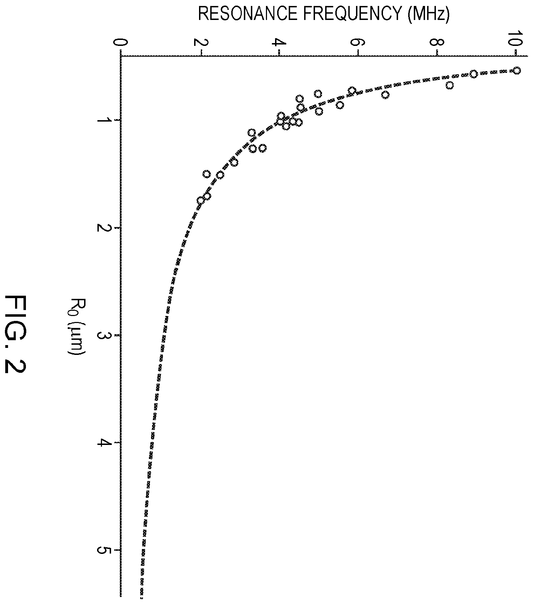

Referring to FIG. 2, the relationship between the microbubble size, Ro, and microbubble resonance frequency may be established prior to the ultrasound procedure. As depicted, in general, the smaller the radius *R*₀ of the microbubbles, the larger will be their resonance frequency. Accordingly, in some embodiments, the resonance frequency of the introduced microbubbles is controlled via their size distribution. For example, after the optimal frequency of the ultrasound is determined as described above, the microbubble radius corresponding to the optimal ultrasound frequency may be computationally interpolated or extrapolated using the established relationship. The size distribution of the microbubbles may then be selected such that a significant fraction (e.g., more than 50%, 90%, 95%, or 99% or more) of the microbubbles have a radius below that corresponding to a resonance frequency equal to the applied ultrasound frequency. In one embodiment, the maximum radius of the administered microbubbles is selected to be at least 50% (or, in some embodiments, a factor by ten) smaller than the radius corresponding to a resonance frequency equal to the applied ultrasound frequency. Approaches for determining and selecting a desired size distribution of microbubbles are provided, for example, in International Application No. PCT/IB2018/000841 (filed on June 29, 2018),

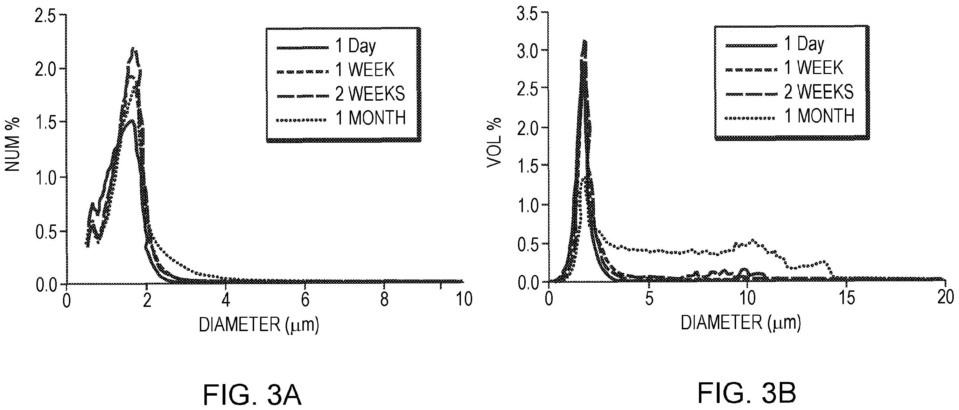

After the desired microbubble size is determined, the microbubbles can be manufactured, or more typically, obtained off-the-shelf. As depicted in FIGS. 3A and 3B, however, commercially available microbubbles are typically polydisperse in size and may undergo ripening during storage. As a result, the actual size of the microbubbles when used may differ from their size when generated and performance may suffer. To solve this problem, in various embodiments, the size of the microbubbles is measured prior to their administration using any suitable approach (such as laser light obscuration and scattering, electrozone sensing, optical microscopy, ultrasound or flow cytometry, all of which are well-known to those of skill in the art and can be implemented without undue experimentation). For example, the ultrasound system 100 may transmit sonications to the microbubbles at multiple frequencies, and based on the attenuation, scattering, backscattering, harmonic generation, or sub-harmonic generation of the transmitted signals and signals from the microbubbles, the size distribution of the microbubbles can be determined. If more than a threshold amount (e.g., 50%, 90%, 95% or 99%) of the microbubbles is no larger than the desired size, the microbubbles may be administered. If, however, less than the threshold amount of the microbubbles is no larger than the desired size, a precautionary action may be taken. For example, the administration system 124 may generate a warning signal to the user.

Additionally, the microbubbles may be centrifuged to isolate the ones conforming to the size criterion. Alternatively, a filter having a pore size of the desired microbubble size may be employed to filter the microbubbles as further described below. In some embodiments, the ultrasound system 100 may be configured to apply an acoustic field to the microbubbles; the acoustic filed has a frequency band and a sufficient amplitude to destroy (e.g., cause cavitation of) the microbubbles that do not conform to the size criterion. After the isolation/filtration/destruction process, the microbubbles may be verified again to ensure that the size thereof matches the desired size; subsequently, the verified microbubbles may be introduced into the patient's body. In one implementation, administration of the microbubbles is synchronized with activation of the ultrasound system 100 and/or administration of complementary materials (such as a therapeutic agent, an MR contrast agent, etc.). For example, upon application of the microbubbles and ultrasound waves to open a target BBB region, the therapeutic agent may be introduced to the target tumor region via the BBB opening to treat the target tumor. Approaches to opening a target BBB region for treating the target tumor are provided, for example, in International Application No. PCT/IB2018/000834 (filed on June 29, 2018),

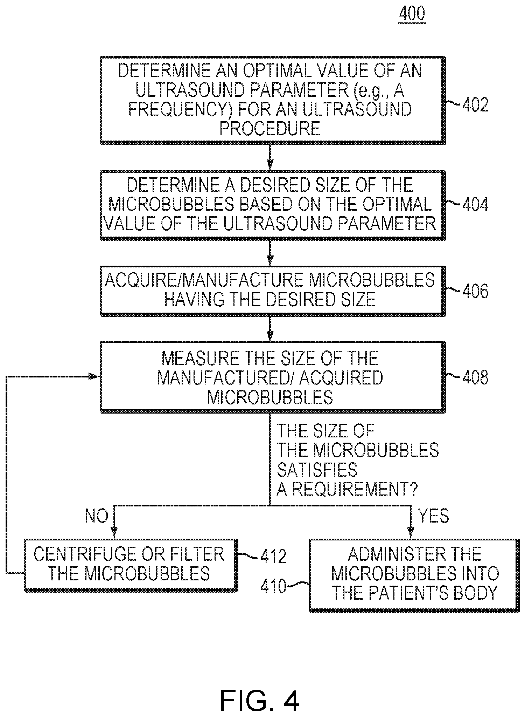

FIG. 4 illustrates a representative procedure 400 for selecting the microbubble size and applying the microbubbles to enhance performance of an ultrasound procedure (e.g., imaging or treatment) in accordance herewith. In a first preparatory step 402, an optimal value of an ultrasound parameter (e.g., frequency) for the procedure is determined based on the characteristics (such as the location, size, shape, type, property, structure, thickness, density, etc.) of the target and/or non-target regions. In one embodiment, the characteristics of the target/non-target regions are acquired using the imager 112 as described above. In a second preparatory step 404, a desired microbubble size for enhancing the performance of the procedure is selected based on the optimal value of the ultrasound parameter. For example, the size distribution of the microbubbles may then be selected such that a significant fraction (e.g., more than 50%, 90%, 95%, or 99% or more) of the microbubbles has a radius substantially below (e.g., a factor of ten lower than) that corresponding to a resonance frequency equal to the applied ultrasound frequency. In a third preparatory step 406, the microbubbles are manufactured or acquired off-the-shelf. In a fourth preparatory step 408, the size of the manufactured/acquired microbubbles is measured using any suitable approach. If the size of the microbubbles satisfies a requirement (e.g., at least 50%, 90%, 95% or 99% of the acquired microbubbles has or is no larger than the desired size), the microbubbles may be administered to enhance the ultrasound imaging quality or treatment effects (step 410). If, however, the microbubbles do not satisfy the size requirement (e.g., less than 50%, 90%, 95% or 99% of the microbubbles has or is no larger than the desired size), the microbubbles may be centrifuged or filtered prior to being introduced into the patient's body (step 412). The size distribution of the centrifuged/filtered microbubbles may be verified again prior to administration.

In some embodiments, the administration profile (e.g., dose, rate, timing or pressure) of the microbubbles is determined based on one or more characteristics (e.g., diameter, size distribution, shell composition, or gas and/or liquid core composition) of the microbubbles prior to treatment. In addition, administration of the microbubbles is synchronized with administration of other materials (e.g., a therapeutic agent or an MR contrast) and/or activation of the ultrasound procedure. For example, after administration of the microbubbles having the desired size distribution into the target region 101, the ultrasound transducer 102 may be activated to perform the ultrasound-mediated, microbubble-enhanced tissue disruption at the target region 101. During the ultrasound procedure, focusing properties (e.g., acoustic power or peak acoustic intensity) of the ultrasound beams and/or the treatment effects (such as tissue-permeability enhancement or ablation) at the target and/or non-target regions may be monitored using the imager 112. In some embodiments, an MRI contrast agent (e.g., a gadolinium-based contrast agent) is injected into the target and/or non-target regions for assisting assessment of the tissue disruption therein. Because of the disrupted tissue, the permeability of the target region may be increased. Accordingly, by monitoring the contrast change in the MRI images, the permeability (and thereby the disruption) of the tissue at the target and/or non-target region can be estimated. In addition, the microbubble response to the applied acoustic field at the target and/or non-target regions may be monitored using the transducer 102 and/or acoustic-signal detector 122.

Based on the monitored treatment effects and/or microbubble response, the controller 108 may adjust the administration profile of the microbubbles and/or ultrasound parameters (e.g., frequencies, amplitudes, phases, activation times) in real time to optimize the treatment or achieve a desired effect at the target. For example, if the treatment effect in the non-target region exceeds a safety threshold and/or an undesired microbubble response is detected and/or an unwanted therapeutic effect at the non-target region is detected, the controller 108 may reduce the administration rate or dose of the microbubbles and/or reduce the amplitude of the sonication waves. Conversely, if the treatment effect in the target region falls below a desired goal, the controller 108 may increase the administration rate or administration time of the microbubbles and/or the sonication amplitude so as to enhance the treatment effect. In addition, the controller 108 may adjust the administration profile of the contrast agent until the treatment effect at the target and/or non-target region can be evaluated. The ultrasound procedure and tissue disruption assessment may be iteratively performed until the volume and/or degree of disrupted tissue substantially matches a desired target volume and/or degree. Approaches to estimating the tissue permeability at the target/non-target regions are provided, for example, in International Application No. PCT/IB2018/000811 (filed on June 29, 2018); approaches to measuring the microbubble response to the applied acoustic field are provided, for example, in International Application No. PCT/IB2018/000774 (filed on May 22, 2018); and approaches to configuring the transducer array for detecting the microbubble response are provided, for example, in U.S. Patent Application No. 62/681,282 (filed on June 6, 2018).

In various embodiments, the ultrasound treatment procedure is performed in combination with other therapeutic methods, such as radiation therapy or targeted drug delivery. For example, the ultrasound-induced microbubble oscillation/cavitation may disrupt vascular tissue in the target region 101; this allows a radiation dose in the radiation therapy to be significantly reduced while still achieving a desired treatment efficacy. In another treatment scenario, the ultrasound-induced microbubble oscillation/cavitation may increase the tissue permeability at the target region; this allows a higher dose of therapeutic agent to reach the target tissue, thereby enhancing the therapeutic effect. Generally, the radiation therapy or targeted drug delivery is performed only after the volume/degree of ultrasound-mediated tissue disruption is verified to match the desired target volume/degree. In addition, different tumors/diseases or different therapeutic agents may require different degrees of tissue disruption mediated by the ultrasound. Accordingly, it is important to operate the transducer 102, imager 112, acoustic-signal detector 122, administration system 124 and/or a radiation device in a controlled manner so as to achieve a desired treatment effect.

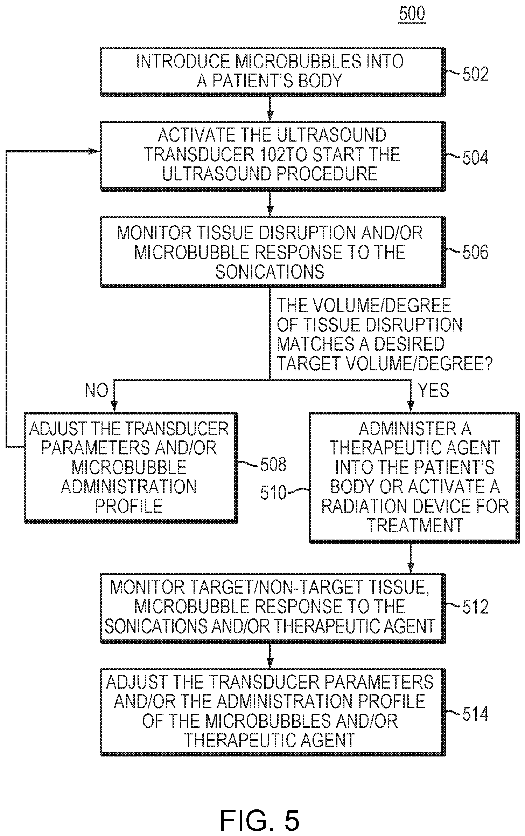

FIG. 5 illustrates a representative procedure 500 for a treatment approach involving the microbubble-enhanced ultrasound procedure in combination with other therapeutic methods (such as radiation therapy or targeted drug delivery) In a first step 502, the microbubbles with characteristics satisfying the predetermined requirements are introduced via the administration system 124 into the patient's body as described above. In a second step 504, the transducer 102 is activated to start the ultrasound-mediated tissue disruption at the target region 101. In addition, the tissue disruption during and/or after the ultrasound procedure may be monitored or assessed using, for example, the imager 112, transducer 102 and/or a contrast agent introduced by the administration system 124 (in a third step 506). Optionally, the microbubble response to the sonications can be monitored using the acoustic-signal detector 122 and/or transducer elements 104. Based on the monitored tissue disruption and/or microbubble response, the transducer parameters and/or microbubble administration profile may be dynamically adjusted until the volume/degree of ultrasound-mediated tissue disruption verifiably matches the desired target volume/degree (in a fourth step 508). The target volume/degree of tissue disruption may vary based on the type, location and/or size of the target region and/or the type and/or size of the therapeutic agent for targeted drug delivery. Steps 504-508 may be iteratively performed throughout the entire ultrasound procedure. The verification is performed by introducing the contrast agent into the target and monitoring the contrast change in the MRI images. Following verification, the therapeutic agent for targeted drug delivery may be introduced via the administration system 124. Alternatively, a radiation device may be activated to start the treatment (in a fifth step 510). Again, the conditions of the target/non-target tissue, microbubbles and/or therapeutic agent may be monitored in real time using, for example, the imager 112, acoustic-signal detector 122 and/or transducer elements 104 (in a sixth step 512); and based thereon, a characteristic (e.g., agent type, size, concentration, administering rate, administering dose, administering pattern, administering time and/or administering pressure) of the microbubbles and/or therapeutic agent can be adjusted to achieved optimal (or desired) treatment effects on the target and/or non-target regions (in a seventh step 514).

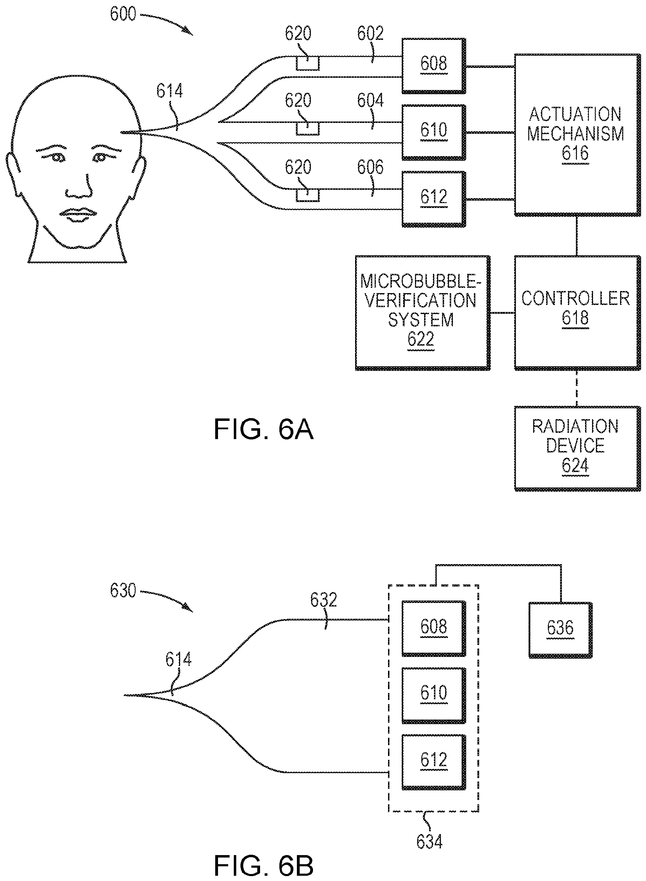

FIG. 6A depicts an exemplary administration system 600 in accordance with various embodiments. The system 600 may include three independent channels 602, 604, 606. The first channel 602 may be in fluid communication with a first container 608 that stores the microbubbles; the second channel 604 may be in fluid communication with a second container 610 that stores the contrast agent; and the third channel 606 may be in fluid communication with a third container 612 that stores the therapeutic agent for targeted drug delivery. The three channels 602, 604, 606 may then be fluidly coupled to an introducing device (e.g., a catheter or a needle) 614 for administering microbubbles, contrast agent and/or therapeutic agent from their corresponding channels into the patient's body. In addition, the system 600 may include an actuation mechanism (e.g., a syringe, a peristaltic pump, etc.) 616 coupled to a controller 618 for forcing the microbubbles, contrast agent and therapeutic agent through their corresponding channels 602, 604, 606 into the introducing device 614 for administration to the patient. Additionally, the administration system 600 may be in communication with a microbubble-verification system 622 that can verify the size of the microbubbles prior to their administration as described above. For example, the microbubble-verification system 622 may include a suitable apparatus for performing laser light obscuration and scattering, electrozone sensing, optical microscopy, ultrasound or flow cytometry, etc.

In some embodiments, the channels 602, 604, 606 and/or introducing device 614 include flow detectors 620 for detecting the flow rate or flow pressure of the fluid therein. In some embodiments, the flow detectors 620 transmit the detected signals to the controller 618 for analysis. In addition, the controller 618 may receive detection signals from the ultrasound transducer 102, imager 122, acoustic-signal detector 122, and/or a radiation device 624 as described above. Based on the received signals, the controller 618 may operate the actuation mechanism 616 for administering the microbubbles, contrast agent and/or therapeutic agent. In addition, the controller 618 may be in communication with the ultrasound controller 108. For example, upon the microbubble-verification system 622 determining that the microbubbles in the container 608 do not conform to the desired size distribution, the controller 618 may cause the ultrasound system 100 to apply an acoustic field onto the microbubbles to destroy microbubbles that do not satisfy the size criterion. Alternatively, the controller 618 may generate a warning signal to the user; the user may then centrifuge and/or filter the microbubbles so as to isolate the microbubbles having the desired size. In addition, the controller 618 may cause activation and deactivation of the transducer 102 based on the received signals from the imager 122, acoustic-signal detector 122, and/or radiation device 628 so as to optimize the treatment effect or achieve a desired treatment effect at the target. The controller 618 may be separate from the ultrasound controller 108 or may be combined with the ultrasound controller 108 into an integrated system control facility.

Accordingly, various embodiments of the present invention advantageously provide an approach that allows the controller 618 and/or the controller 108 to adjust the microbubble administration and/or ultrasound sonication in real time during the ultrasound procedure so as to achieve a desired tissue disruption effect. The tissue disruption effect may be verified using the contrast agent or other suitable approach. Finally, after the tissue disruption effect is verified, the target treatment may be performed by administering the therapeutic agent or activating a radiation device.

Although the system described above has three channels, one of ordinary skill in the art will understand that systems may have different numbers of channels that ultimately terminate in the introducing device 614 and are within the scope of the current invention. For example, fewer independently controlled fluid channels may be utilized in the current invention. Referring to FIG. 6B, the administration system 630 may include a single channel 632; the three containers 608, 610, and 612 may be placed on a moving tray 634. By shifting the relative locations of the containers with respect to the channel 632, the microbubbles, contrast agent and therapeutic agent stored in the containers 608, 610, 612, respectively, may be independently and separately injected into the channel 632. In addition, the administration system 630 may include a preparation device 636 for preparing the microbubbles, contrast agent and/or therapeutic agent prior to administration. For example, the preparation device 636 may include a heater for heating the fluid to a predetermined temperature (e.g., body temperature) prior to administration. In some embodiments, the preparation device 636 includes a shaker for shaking the container(s) so as to mix the microbubbles, contrast agent and/or therapeutic agent prior to injection.

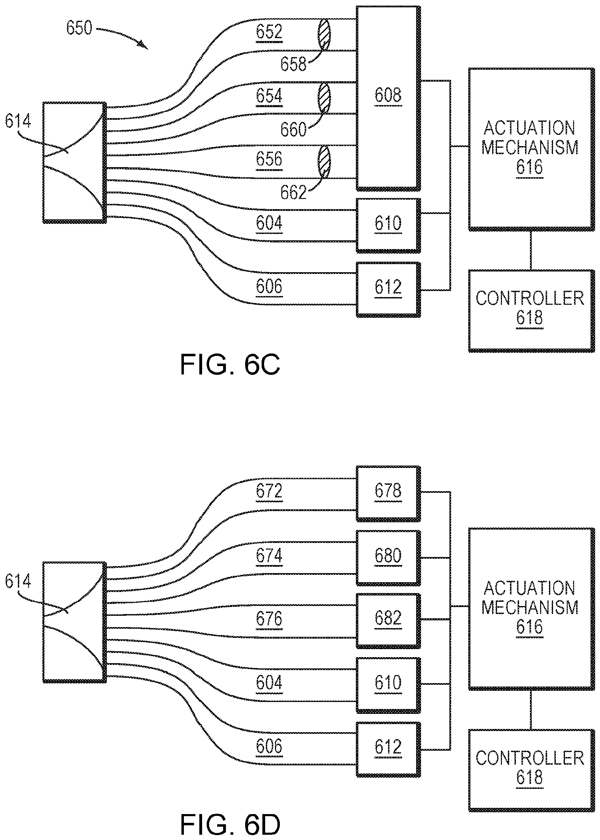

In addition, each container may be fluidly coupled to more than one channel. Referring to FIG. 6C, in some embodiments, the administration system 650 includes multiple channels 652, 654, 656 fluidically connected to the microbubble vial 608 having therein, for example, microbubbles polydisperse in size; each channel has a filter of a different pore size. For example, the channels 652, 654, 656 may have filters 658, 660, 662 with pore sizes of 0.5 µm, 2 µm and 5 µm, respectively. By coupling the vial 608 to different channels, microbubbles having different maximum sizes may be generated. This approach is particularly useful when the condition (e.g., size or temperature) of the target region 101 and/or non-target region and/or microbubble response to the ultrasound waves changes during treatment, and dynamic adjustment of the microbubble size is desired to maintain optimal treatment effects at the target region 101 while minimizing damage to the non-target region. In one implementation, the fluid coupling between the vial 608 and the channels 652-656 can be adjusted in real time based on the monitored target/non-target condition and/or microbubble response as described above.

In various embodiments, more than one container may be utilized for handling each of the microbubbles, contrast agent, and/or therapeutic agent. For example, referring to FIG. 6D, each of the channels 672, 674, 676 may be coupled to a different microbubble vial having microbubbles of a different size. For example, the channels 672, 674, 676 may be coupled to vials 678, 680, 682, in which at least 50%, 80%, 90%, or 95% of the microbubbles have a radius of 0.5 µm, 2 µm and 5 µm, respectively. The actuation mechanism 616 may separately force the microbubbles in each vial through a corresponding channel to enter the introducing device 614, via which they are administered to the patient. Alternatively, each channel may be coupled to a separate actuation mechanism. Again, during the ultrasound procedure, based on the monitored condition of the target and/or non-target regions and/or the monitored microbubble response, the controller 618 may cause the actuation mechanism 616 to administer microbubbles having the desired size from the vial into the patient's body so as to optimize the imaging quality or treatment effect on the target region. For example, when the treatment effect in the non-target region exceeds the safety threshold and/or an undesired microbubble response and/or therapeutic effect at the non-target region is detected, the controller 618 may cause the actuation mechanism 616 to administer microbubbles from the vial containing microbubbles whose size corresponds to a resonance frequency that differs further from the ultrasound frequency. In situations where the temperature in the target region is sufficiently below the desired ablation temperature, the controller 618 may cause the actuation mechanism 616 to administer microbubbles from the vial containing microbubbles whose size corresponds to a resonance frequency that is closer to the ultrasound frequency.

Accordingly, various embodiments of the present invention advantageously provide an approach that allows the size and/or administration profile of microbubbles to be verified prior to the ultrasound procedure and adjusted in real time during the ultrasound procedure so as to maximize the performance of the procedure and ensure patient safety.

It should be noted although the ultrasound procedure described herein is enhanced using microbubbles, the systems and methods described above may also be implemented for ultrasound procedures enhanced using other approaches. For example, emulsions and/or droplets composed of various liquid perfluorocarbon agents may be utilized to enhance the ultrasound procedure. Accordingly, the administration system described herein may be used to introduce the emulsions/droplets into the target region, and the approaches for measuring and manipulating various characteristics of the microbubbles may be applied to measure and manipulate the characteristics (e.g., the size, shell composition (if any), liquid core composition, etc.) of the emulsions/droplets as well.

The therapeutic agent may include any drug that is suitable for treating a tumor. For example, for treating glioblastoma (GBM), the drug may include or consist of, e.g., one or more of Busulfan, Thiotepa, CCNU (lomustine), BCNU (carmustine), ACNU (nimustine), Temozolomide, Methotrexate, Topotecan, Cisplatin, Etoposide, Irinotecan /SN-38, Carboplatin, Doxorubicin, Vinblastine, Vincristine, Procarbazine, Paclitaxel, Fotemustine, Ifosfamide /4-Hydroxyifosfamide /aldoifosfamide, Bevacizumab, 5-Fluorouracil, Bleomycin, Hydroxyurea, Docetaxel, Cytarabine (cytosine arabinoside, ara-C) /ara-U, etc.

In addition, for treating GBM, those skilled in the art can select a drug and a BBB opening regime optimized to enhance drug absorption across the BBB within patient safety constraints. In this regard, it is known that the BBB is actually already disrupted in the core of many tumors, allowing partial penetration of antitumor drugs; but the BBB is widely intact around the "brain adjacent to tumor" (BAT) region where invasive/escaping GBM cells can be found, and which cause tumor recurrence. Overcoming the BBB for better drug delivery within the tumor core and the BAT can be accomplished using ultrasound as described herein. The drugs employed have various degrees of toxicity and various penetration percentages through the BBB. An ideal drug has high cytotoxicity to the tumor and no BBB penetration (so that its absorption and cytotoxic effects can be confined to regions where the BBB is disrupted), low neurotoxicity (to avoid damage to the nervous system), and tolerable systemic toxicity (e.g., below a threshold) at the prescribed doses. The drug may be administered intravenously or, in some cases, by injection proximate to the tumor region.

In general, functionality for utilizing microbubbles to enhance ultrasound treatment and/or targeted drug delivery while limiting damage to non-target tissue may be structured in one or more modules implemented in hardware, software, or a combination of both, whether integrated within a controller of the imager 112, an ultrasound system 100 and/or the administration system 124, or provided by a separate external controller or other computational entity or entities. Such functionality may include, for example, analyzing imaging data of the target and/or non-target regions acquired using the imager 112, determining the optimal value(s) of ultrasound parameter(s) empirically and/or using the imaging data, establishing a relationship between a microbubble size distribution and a microbubble resonance frequency, determining a microbubble resonance frequency and its corresponding microbubble size distribution based on the optimal ultrasound parameter value(s), causing the size of the microbubbles to be verified using any suitable approach, causing the microbubbles to be centrifuged, filtered or otherwise fractionated to isolate the microbubbles having the desired size, causing the ultrasound system 100 to apply an acoustic field onto the microbubbles so as to destroy (e.g., cause cavitation thereof) the ones that do not conform to the size criterion, causing the microbubble having the verified size distribution to be introduced to the target region 101, causing the ultrasound transducer to be activated using the determine optimal parameter value(s), monitoring the therapeutic effect and/or microbubble response at the target/non-target regions during the ultrasound procedure, and/or adjusting the optimal ultrasound parameter value(s) and/or microbubble resonance frequency as described above.

In addition, the ultrasound controller 108 and/or the administration system controller 618 may include one or more modules implemented in hardware, software, or a combination of both. For embodiments in which the functions are provided as one or more software programs, the programs may be written in any of a number of high level languages such as PYTHON, FORTRAN, PASCAL, JAVA, C, C++, C#, BASIC, various scripting languages, and/or HTML. Additionally, the software can be implemented in an assembly language directed to the microprocessor resident on a target computer; for example, the software may be implemented in Intel 80x86 assembly language if it is configured to run on an IBM PC or PC clone. The software may be embodied on an article of manufacture including, but not limited to, a floppy disk, a jump drive, a hard disk, an optical disk, a magnetic tape, a PROM, an EPROM, EEPROM, field-programmable gate array, or CD-ROM. Embodiments using hardware circuitry may be implemented using, for example, one or more FPGA, CPLD or ASIC processors.

The invention is defined in the following claims. Other embodiments, examples, equivalents and, in particular, methods of treatment, are not a part of the invention.

## Claims

1. A system for microbubble-enhanced treatment of target tissue, the system comprising:
(i) an administration device comprising a first storage container for storing microbubbles, wherein the first storage container is connected to a plurality of channels, each having a manipulation means for changing the microbubble characteristic, and a controller configured to:
(a) receive a desired characteristic of the microbubbles for treating the target tissue;
(b) cause the microbubbles to be dispensed from the administration device; and
(c) compare a characteristic of the dispensed microbubbles to the desired
characteristic so as to validate a match therebetween; **characterised in that** the manipulation means is configured to apply an acoustic field to the microbubbles;
OR
(ii) an administration device comprising a storage container for storing microbubbles; and
a controller configured to:
(a) receive a desired characteristic of the microbubbles for treating the target tissue; and
(b) cause substantially only the microbubbles having the desired characteristic to be administered into the target tissue,
further comprising:
at least one fluidic channel coupled to the storage container for delivering the microbubbles to the target tissue; and
a manipulator associated with the at least one fluidic channel for changing therein the microbubble characteristic,
**characterised in that**
the manipulator comprises an ultrasound transducer for applying an acoustic field to the microbubbles in the at least one fluidic channel.

2. The system of claim 1(i), wherein the controller is further configured to: (a) cause the dispensed microbubbles to be introduced to the target tissue upon validation of the match; or (b) cause a precautionary action upon detecting a clinically significant deviation between the characteristic of the dispensed microbubbles from the desired characteristic.

3. The system of claim 1(i), further comprising a measurement system for measuring the characteristic of the dispensed microbubbles.

4. The system of claim 3, wherein the measurement system comprises an acoustic system, optionally
wherein the acoustic system is configured to: (a) apply a plurality of frequencies to the microbubbles for measuring the characteristic thereof; or
(b) measure at least one of attenuation, scattering, backscattering, harmonic generation, or sub-harmonic generation from the microbubbles.

5. The system of claim 3, wherein the measurement system: (a) is configured to perform measurements of at least one of a laser light obscuration and scattering, electrozone sensing, optical microscopy or flow cytometry;
(b) comprises at least one sensor for measuring an administration rate of the microbubbles; or
(c) further comprises an ultrasound transducer for transmitting ultrasound waves to the microbubbles, wherein the measurement system is configured to monitor a response of the microbubbles to the ultrasound waves, optionally
wherein the controller is further configured to adjust at least one of an ultrasound parameter or the characteristic of the dispensed microbubbles based at least in part on the monitored response thereof, further optionally
wherein the ultrasound parameter comprises at least one of a frequency, a phase, or an amplitude of a signal driving the ultrasound transducer.

6. The system of claim 1(i), further comprising an ultrasound transducer, wherein the administration device further comprises a second storage container for storing a therapeutic agent and the controller is further configured to:
cause the ultrasound transducer to transmit treatment ultrasound waves to the target tissue and generate an acoustic field therein; and
cause administration of the therapeutic agent so as to produce an optimal treatment effect in the target tissue.

7. The system of claim 6, further comprising a measurement system for detecting signals indicating a condition of a treatment procedure.

8. The system of claim 7, wherein the condition of the treatment procedure comprises at least one of a condition of the target tissue, a condition of the therapeutic agent, or a condition of the microbubbles, optionally
wherein the condition of: (a) the microbubbles is a cavitation state thereof; or
(b) the target tissue comprises at least one of a temperature, a size, a shape or a location of the target tissue.

9. The system of claim 7, wherein: (a) the measurement system comprises at least one of an imager or an acoustic-signal detector; or
(b) the controller is further configured to adjust the characteristic of at least one of the administered microbubbles or the administered therapeutic agent based at least in part on the detected signals, optionally
wherein the characteristic is at least one of an agent type, a size, a concentration, an administering rate, an administering dose, an administering pattern, an administering time or an administering pressure of the administered microbubbles and/or therapeutic agent.

10. The system of claim 6, wherein: (a) the controller is further configured to cause the ultrasound transducer to transmit ultrasound waves to the target tissue in the presence of the administered microbubbles;
(b) the administration device further comprises a third storage container for storing a contrast agent, optionally
wherein the administration device further comprises:
an actuation mechanism;
an introducing device for delivering at least one of the microbubbles, therapeutic agent, or contrast agent to the target tissue; and
at least one channel for delivering at least one of the microbubbles, contrast agent or therapeutic agent from the corresponding storage container to the introducing device; or
(c) the therapeutic agent comprises at least one of Busulfan, Thiotepa, CCNU (lomustine), BCNU (carmustine), ACNU (nimustine), Temozolomide, Methotrexate, Topotecan, Cisplatin, Etoposide, Irinotecan /SN-38, Carboplatin, Doxorubicin, Vinblastine, Vincristine, Procarbazine, Paclitaxel, Fotemustine, Ifosfamide /4-Hydroxyifosfamide /aldoifosfamide, Bevacizumab, 5-Fluorouracil, Bleomycin, Hydroxyurea, Docetaxel, or Cytarabine (cytosine arabinoside, ara-C)/ara-U.

11. The system of claim 1(i), wherein: (a) the manipulation means comprises a plurality of filters having different exclusion sizes, the controller being configured to:
select a channel having one of the filters corresponding to the desired microbubble characteristic; and
cause the microbubbles to be administered only through the selected channel; or (b) the controller is further configured to tune a frequency band of the acoustic field so as to destroy the microbubbles having a size outside a desired size range.

12. The system of claim 1(i), further comprising: (a) a plurality of storage containers, each containing microbubbles having a different size characteristic, the controller being configured to:
select one of the storage containers having therein microbubbles corresponding to the desired microbubble characteristic; and
cause the microbubbles to be administered only from the selected storage container; or
(b) a radiation device for transmitting a radiation dose to the target tissue.

13. The system of claim 1(i), wherein the controller is further configured to dispense the microbubbles from the administration device based at least in part on a predetermined administration profile.

14. The system of claim 13, wherein the administration profile: (a) is determined based at least in part on the characteristic of the dispensed microbubbles, optionally wherein the characteristic of the dispensed microbubbles comprises at least one of a diameter, a size distribution, a shell composition, a gas composition or a liquid core composition; or (b) comprises at least one of a dose, a concentration, an administering rate, an administering timing or an administering pressure of the microbubbles.

15. The system of claim 1(i), further comprising: (a) a preparation device for preparing the microbubbles based at least in part on the desired characteristic; or
(b) wherein the controller is further configured to acquire a characteristic of the target tissue and determine the desired characteristic of the microbubbles based at least in part on the characteristic of the target tissue.

16. The system of claim 1(ii), wherein: (a) the manipulator comprises a filter having a determined size characteristic;
(b) the transducer is configured to apply an acoustic field having at least one frequency band so as to destroy the microbubbles having a size outside a desired size range; or
(c) the controller is further configured to acquire a characteristic of the target tissue and determine the desired characteristic of the microbubbles based at least in part on the characteristic of the target tissue.

17. The system of claim 1(ii), wherein the controller is further configured to administer the microbubbles based on a predetermined administration profile.

18. The system of claim 17, wherein the administration profile: (a) is determined based at least in part on a second characteristic of the microbubbles, optionally
wherein the second characteristic of the microbubbles comprises at least one of a diameter, a size distribution, a shell composition, or a gas and/or liquid core composition; or
(b) comprises at least one of a dose, a concentration, an administering rate, an administering timing or an administering pressure of the microbubbles.

19. The system of claim 1(ii), further comprising a preparation device for preparing the microbubbles based at least in part on the desired characteristic, substantially all of the prepared microbubbles having the desired characteristic.

## Patentansprüche

1. System zur mikroblasenverstärkten Behandlung von Zielgewebe, wobei das System Folgendes umfasst:
(i) eine Verabreichungsvorrichtung, umfassend einen ersten Aufbewahrungsbehälter zum Aufbewahren von Mikroblasen, wobei der erste Aufbewahrungsbehälter mit einer Vielzahl von Kanälen verbunden ist, die jeweils ein Manipulationsmittel zum Ändern der Mikroblaseneigenschaft aufweisen, und
eine Steuerung, die konfiguriert ist zum:
(a) Empfangen einer gewünschten Eigenschaft der Mikroblasen zum Behandeln des Zielgewebes;
(b) Veranlassen, dass die Mikroblasen von der Verabreichungsvorrichtung ausgegeben werden; und
(c) Vergleichen einer Eigenschaft der abgegebenen Mikroblasen mit der gewünschten Eigenschaft, um eine Übereinstimmung dazwischen zu validieren;
**dadurch gekennzeichnet, dass** das Manipulationsmittel dazu konfiguriert ist, ein Schallfeld an die Mikroblasen anzulegen;
ODER
(ii) eine Verabreichungsvorrichtung, umfassend einen Aufbewahrungsbehälter zum Aufbewahren von Mikroblasen; und
eine Steuerung, die konfiguriert ist zum:
(a) Empfangen einer gewünschten Eigenschaft der Mikroblasen zum Behandeln des Zielgewebes; und
(b) Veranlassen, dass im Wesentlichen nur die Mikroblasen mit der gewünschten Eigenschaft in das Zielgewebe verabreicht werden,
ferner umfassend:
mindestens einen Fluidkanal, der mit dem Aufbewahrungsbehälter gekoppelt ist, zum Abgeben der Mikroblasen an das Zielgewebe; und
einen Manipulator, der mit dem mindestens einen Fluidkanal assoziiert ist, zum Ändern der Mikroblaseneigenschaft darin,
**dadurch gekennzeichnet, dass**
der Manipulator einen Ultraschallwandler zum Anlegen eines Schallfelds an die Mikroblasen in dem mindestens einen Fluidkanal umfasst.

2. System nach Anspruch 1(i), wobei die Steuerung ferner konfiguriert ist zum: (a) Veranlassen, dass die ausgegebenen Mikroblasen nach der Validierung der Übereinstimmung in das Zielgewebe eingebracht werden; oder (b) Veranlassen eine vorbeugenden Maßnahme nach Detektieren einer klinisch signifikanten Abweichung zwischen der Eigenschaft der ausgegebenen Mikroblasen von der gewünschten Eigenschaft.

3. System nach Anspruch 1(i), ferner umfassend ein Messsystem zum Messen der Eigenschaft der ausgegebenen Mikroblasen.

4. System nach Anspruch 3, wobei das Messsystem ein Schallsystem umfasst, optional
wobei das Schallsystem konfiguriert ist zum: (a) Anlegen einer Vielzahl von Frequenzen an die Mikroblasen zum Messen der Eigenschaft davon; oder
(b) Messen von mindestens einer von Dämpfung, Streuung, Rückstreuung, harmonischer Erzeugung oder subharmonischer Erzeugung von den Mikroblasen.

5. System nach Anspruch 3, wobei das Messsystem: (a) dazu konfiguriert ist, Messungen von mindestens einer von Laserlichtverdunkelung und -streuung, Elektrozonenerfassung, optischer Mikroskopie oder Durchflusszytometrie durchzuführen;
(b) mindestens einen Sensor zum Messen einer Verabreichungsrate der Mikroblasen umfasst; oder
(c) ferner einen Ultraschallwandler zum Übertragen von Ultraschallwellen an die Mikroblasen umfasst, wobei das Messsystem dazu konfiguriert ist, eine Reaktion der Mikroblase auf die Ultraschallwellen zu überwachen, optional
wobei die Steuerung ferner dazu konfiguriert ist, mindestens eines von einem Ultraschallparameter oder der Eigenschaft der ausgegebenen Mikroblasen basierend mindestens teilweise auf der überwachten Reaktion davon anzupassen, ferner optional
wobei der Ultraschallparameter mindestens eine von einer Frequenz, einer Phase oder einer Amplitude eines Signals umfasst, das den Ultraschallwandler antreibt.

6. System nach Anspruch 1(i), ferner umfassend einen Ultraschallwandler, wobei die Verabreichungsvorrichtung ferner einen zweiten Aufbewahrungsbehälter zum Aufbewahren eines therapeutischen Mittels umfasst und die Steuerung ferner konfiguriert ist zum:
Veranlassen, dass der Ultraschallwandler Behandlungsultraschallwellen an das Zielgewebe überträgt und darin ein Schallfeld erzeugt; und
Veranlassen der Verabreichung des therapeutischen Mittels, um eine optimale Behandlungswirkung im Zielgewebe zu produzieren.

7. System nach Anspruch 6, ferner umfassend ein Messsystem zum Detektieren von Signalen, die einen Zustand einer Behandlungsprozedur angeben.

8. System nach Anspruch 7, wobei der Zustand der Behandlungsprozedur mindestens einen von einem Zustand des Zielgewebes, einem Zustand des therapeutischen Mittels oder einem Zustand der Mikroblasen umfasst, optional
wobei der Zustand von: (a) den Mikroblasen ein Kavitationszustand davon ist; oder (b) dem Zielgewebe mindestens eines von einer Temperatur, einer Größe, einer Form oder einem Ort des Zielgewebes umfasst.

9. System nach Anspruch 7, wobei: (a) das Messsystem mindestens einen von einem Bildgeber oder einen Schallsignaldetektor umfasst; oder
(b) die Steuerung ist ferner dazu konfiguriert ist, die Eigenschaft von mindestens einem von den verabreichten Mikroblasen oder dem verabreichten therapeutischen Mittel mindestens teilweise basierend auf den detektierten Signalen anzupassen, optional
wobei die Eigenschaft mindestens eines von einem Mitteltyp, einer Größe, einer Konzentration, einer Verabreichungsrate, einer Verabreichungsdosis, einem Verabreichungsmuster, einer Verabreichungszeit oder einem Verabreichungsdruck der verabreichten Mikroblasen und/oder des verabreichten therapeutischen Mittels ist.

10. System nach Anspruch 6, wobei: (a) die Steuerung ferner dazu konfiguriert ist, zu veranlassen, dass der Ultraschallwandler in der Gegenwart der verabreichten Mikroblasen Ultraschallwellen an das Zielgewebe überträgt;
(b) die Verabreichungsvorrichtung ferner einen dritten Aufbewahrungsbehälter zum Aufbewahren eines Kontrastmittels umfasst, optional
wobei die Verabreichungsvorrichtung ferner Folgendes umfasst:
einen Betätigungsmechanismus;
eine Einbringungsvorrichtung zum Abgeben von mindestens einem von den Mikroblasen, dem therapeutischen Mittel oder dem Kontrastmittel an das Zielgewebe; und
mindestens einen Kanal zum Abgeben von mindestens einem von den Mikroblasen, dem Kontrastmittel oder dem therapeutischen Mittel aus dem entsprechenden Aufbewahrungsbehälter an die Einbringungsvorrichtung; oder
(c) das therapeutische Mittel mindestens eines von Busulfan, Thiotepa, CCNU (Lomustin), BCNU (Carmustin), ACNU (Nimustin), Temozolomid, Methotrexat, Topotecan, Cisplatin, Etoposid, Irinotecan/SN-38, Carboplatin, Doxorubicin, Vinblastin, Vincristin, Procarbazin, Paclitaxel, Fotemustin, Ifosfamid/4-Hydroxyifosfamid/Aldoifosfamid, Bevacizumab, 5-Fluorouracil, Bleomycin, Hydroxyharnstoff, Docetaxel oder Cytarabin (Cytosinarabinosid, ara-C)/ara-U umfasst.

11. System nach Anspruch 1(i), wobei: (a) das Manipulationsmittel eine Vielzahl von Filtern mit unterschiedlichen Ausschlussgrößen umfasst, wobei die Steuerung konfiguriert ist zum:
Auswählen eines Kanals mit einem der Filter entsprechend der gewünschten Mikroblaseneigenschaft; und
Veranlassen, dass die Mikroblasen nur durch den ausgewählten Kanal verabreicht werden; oder
(b) die Steuerung ferner dazu konfiguriert ist, ein Frequenzband des Schallfelds anzupassen, um die Mikroblasen mit einer Größe außerhalb eines gewünschten Größenbereichs zu zerstören.

12. System nach Anspruch 1(i), ferner umfassend: (a) eine Vielzahl von Aufbewahrungsbehältern, die jeweils Mikroblasen mit einer anderen Größeneigenschaft aufweisen, wobei die Steuerung konfiguriert ist zum:
Auswählen eines der Aufbewahrungsbehälter mit Mikroblasen darin entsprechend der gewünschten Mikroblaseneigenschaft; und
Veranlassen, dass die Mikroblasen nur aus dem ausgewählten Aufbewahrungsbehälter verabreicht werden;
oder
(b) eine Bestrahlungsvorrichtung zum Übertragen einer Bestrahlungsdosis an das Zielgewebe.

13. System nach Anspruch 1(i), wobei die Steuerung ferner dazu konfiguriert ist, die Mikroblasen mindestens teilweise basierend auf einem vorbestimmten Verabreichungsprofil aus der Verabreichungsvorrichtung auszugeben.

14. System nach Anspruch 13, wobei das Verabreichungsprofil: (a) mindestens teilweise basierend auf der Eigenschaft der ausgegebenen Mikroblasen bestimmt wird, optional
wobei die Eigenschaft der ausgegebenen Mikroblasen mindestens eines von einem Durchmesser, einer Größenverteilung, einer Hüllenzusammensetzung, einer Gaszusammensetzung oder einer Flüssigkernzusammensetzung umfasst; oder
(b) mindestens eines von einer Dosis, einer Konzentration, einer Verabreichungsrate, einem Verabreichungszeitpunkt oder einem Verabreichungsdruck der Mikroblasen umfasst.

15. System nach Anspruch 1(i), ferner umfassend: (a) eine Herstellungsvorrichtung zum Herstellen der Mikroblasen mindestens teilweise basierend auf der gewünschten Eigenschaft; oder
(b) wobei die Steuerung ferner dazu konfiguriert ist, eine Eigenschaft des Zielgewebes zu erlangen und die gewünschte Eigenschaft der Mikroblasen mindestens teilweise basierend auf der Eigenschaft des Zielgewebes zu bestimmen.

16. System nach Anspruch 1 (ii), wobei: (a) der Manipulator einen Filter mit einer bestimmten Größeneigenschaft umfasst;
(b) der Wandler dazu konfiguriert ist, ein Schallfeld mit mindestens einem Frequenzband anzulegen, um die Mikroblasen mit einer Größe außerhalb eines gewünschten Größenbereichs zu zerstören; oder
(c) die Steuerung ferner dazu konfiguriert ist, eine Eigenschaft des Zielgewebes zu erlangen und die gewünschte Eigenschaft der Mikroblasen mindestens teilweise basierend auf der Eigenschaft des Zielgewebes zu bestimmen.

17. System nach Anspruch 1(ii), wobei die Steuerung ferner dazu konfiguriert ist, die Mikroblasen basierend auf einem vorbestimmten Verabreichungsprofil zu verabreichen.

18. System nach Anspruch 17, wobei das Verabreichungsprofil: (a) mindestens teilweise basierend auf einer zweiten Eigenschaft der Mikroblasen bestimmt wird, optional
wobei die zweite Eigenschaft der Mikroblasen mindestens eines von einem Durchmesser, einer Größenverteilung, einer Hüllenzusammensetzung oder einer Gas- und/oder Flüssigkernzusammensetzung umfasst; oder
(b) mindestens eines von einer Dosis, einer Konzentration, einer Verabreichungsrate, einem Verabreichungszeitpunkt oder einem Verabreichungsdruck der Mikroblasen umfasst.

19. System nach Anspruch 1(ii), ferner umfassend eine Herstellungsvorrichtung zum Herstellen der Mikroblasen mindestens teilweise basierend auf der gewünschten Eigenschaft, wobei im Wesentlichen alle der hergestellten Mikroblasen die gewünschte Eigenschaft aufweisen.

## Revendications

1. Système destiné au traitement renforcé par des microbulles d'un tissu cible, le système comprenant :
(i) un dispositif d'administration comprenant un premier réservoir de stockage destiné à stocker des microbulles, ledit premier réservoir de stockage étant relié à une pluralité de canaux, comportant chacun un moyen de manipulation pour modifier la caractéristique des microbulles, et
un dispositif de commande conçu pour :
(a) recevoir une caractéristique souhaitée des microbulles pour traiter le tissu cible ;
(b) provoquer la distribution des microbulles depuis le dispositif d'administration ; et
(c) comparer une caractéristique des microbulles distribuées à la caractéristique souhaitée de façon à valider une correspondance entre elles ;
**caractérisé en ce que** le moyen de manipulation est conçu pour appliquer un champ acoustique sur les microbulles ;
OU
(ii) un dispositif d'administration comprenant un réservoir de stockage destiné à stocker des microbulles ; et
un dispositif de commande conçu pour :
(a) recevoir une caractéristique souhaitée des microbulles pour traiter le tissu cible ; et
(b) provoquer sensiblement l'administration de seulement les microbulles présentant la caractéristique souhaitée dans le tissu cible,
comprenant en outre :
au moins un canal fluidique relié au réservoir de stockage pour distribuer les microbulles au tissu cible ; et
un manipulateur associé audit au moins un canal fluidique pour modifier en son sein la caractéristique des microbulles,
**caractérisé en ce que**
le manipulateur comprend un transducteur à ultrasons pour appliquer un champ acoustique sur les microbulles dans ledit au moins un canal fluidique.

2. Système selon la revendication 1(i), ledit dispositif de commande étant en outre conçu pour : (a) provoquer l'introduction des microbulles distribuées dans le tissu cible lors de la validation de la correspondance ; ou
(b) provoquer une mesure de précaution lors de la détection d'un écart cliniquement significatif entre la caractéristique des microbulles distribuées par rapport à la caractéristique souhaitée.

3. Système selon la revendication 1(i), comprenant en outre un système de mesure destiné à mesurer la caractéristique des microbulles distribuées.

4. Système selon la revendication 3, ledit système de mesure comprenant un système acoustique, éventuellement
ledit système acoustique étant conçu pour : (a) appliquer une pluralité de fréquences sur les microbulles pour mesurer la caractéristique de celles-ci ; ou
(b) mesurer au moins une caractéristique parmi l'atténuation, la diffusion, la rétrodiffusion, la génération d'harmoniques ou la génération de sous-harmoniques à partir des microbulles.

5. Système selon la revendication 3, ledit système de mesure : (a) étant conçu pour réaliser des mesures d'au moins l'une parmi l'occultation et la diffusion d'une lumière laser, la détection d'électrozone, la microscopie optique ou la cytométrie de flux ;
(b) comprenant au moins un détecteur pour mesurer la vitesse d'administration des microbulles ; ou
(c) comprenant en outre un transducteur à ultrasons pour transmettre des ondes ultrasonores aux microbulles, ledit système de mesure étant conçu pour surveiller une réaction des microbulles aux ondes ultrasonores, éventuellement
ledit dispositif de commande étant en outre conçu pour régler au moins un élément parmi un paramètre des ultrasons ou la caractéristique des microbulles distribuées en fonction au moins en partie de la réaction surveillée de celles-ci, éventuellement en outre
ledit paramètre des ultrasons comprenant au moins l'un parmi une fréquence, une phase ou une amplitude d'un signal commandant le transducteur à ultrasons.

6. Système selon la revendication 1(i), comprenant en outre un transducteur à ultrasons, ledit dispositif d'administration comprenant en outre un deuxième réservoir de stockage destiné à stocker un agent thérapeutique et ledit dispositif de commande étant en outre conçu pour :
amener le transducteur à ultrasons à transmettre des ondes ultrasonores de traitement au tissu cible et à générer un champ acoustique en son sein ; et
provoquer l'administration de l'agent thérapeutique de façon à produire un effet de traitement optimal dans le tissu cible.

7. Système selon la revendication 6, comprenant en outre un système de mesure destiné à détecter des signaux indiquant un état d'une procédure de traitement.

8. Système selon la revendication 7, ledit état de la procédure de traitement comprenant au moins un état parmi un état du tissu cible, un état de l'agent thérapeutique ou un état des microbulles, éventuellement
ledit état : (a) des microbulles étant un état de cavitation de celles-ci ; ou
(b) le tissu cible comprenant au moins l'un parmi une température, une taille, une forme ou un emplacement du tissu cible.

9. Système selon la revendication 7 : (a) ledit système de mesure comprenant au moins l'un parmi un imageur et un détecteur de signaux acoustiques ; ou
(b) ledit dispositif de commande étant en outre conçu pour régler la caractéristique d'au moins un élément parmi les microbulles administrées ou l'agent thérapeutique administré en fonction au moins en partie des signaux détectés, éventuellement
ladite caractéristique étant au moins l'une parmi un type d'agent, une taille, une concentration, une vitesse d'administration, une dose d'administration, un schéma d'administration, un moment d'administration ou une pression d'administration des microbulles et/ou de l'agent thérapeutique administrés.

10. Système selon la revendication 6 : (a) ledit dispositif de commande étant en outre conçu pour amener le transducteur à ultrasons à transmettre des ondes ultrasonores au tissu cible en présence des microbulles administrées ;
(b) ledit dispositif d'administration comprenant en outre un troisième réservoir de stockage destiné à stocker un agent de contraste, éventuellement
ledit dispositif d'administration comprenant en outre :
un mécanisme d'actionnement ;
un dispositif d'introduction destiné à distribuer au moins l'un parmi les microbulles, l'agent thérapeutique ou l'agent de contraste au tissu cible ; et
au moins un canal destiné à distribuer au moins un élément parmi les microbulles, l'agent de contraste ou l'agent thérapeutique du réservoir de stockage correspondant au dispositif d'introduction ; ou
(c) ledit agent thérapeutique comprenant au moins l'un parmi le busulfan, le thiotépa, la CCNU (lomustine), la BCNU (carmustine), l'ACNU (nimustine), le témozolomide, le méthotrexate, le topoécan, le cisplatine, l'étoposide, l'irinotécan/SN-38, le carboplatine, la doxorubicine, la vinblastine, la vincristine, la procarbazine, le paclitaxel, la fotémustine, l'ifosfamide/4-hydroxyifosfamide/aldoifosfamide, le bevacizumab, le 5-fluorouracile, la bléomycine, l'hydroxyurée, le docétaxel ou la cytarabine (cytosine arabinoside, ara-C)/ara-U.

11. Système selon la revendication 1(i) : (a) ledit moyen de manipulation comprenant une pluralité de filtres présentant différentes tailles d'exclusion, ledit dispositif de commande étant conçu pour :
sélectionner un canal comportant l'un des filtres correspondant à la caractéristique des microbulles souhaitée ; et
provoquer l'administration des microbulles seulement par l'intermédiaire du canal sélectionné ; ou
(b) ledit dispositif de commande étant en outre conçu pour syntoniser une bande de fréquences du camp acoustique de façon à détruire les microbulles présentant une taille en dehors d'une plage de tailles souhaitée.

12. Système selon la revendication 1(i), comprenant en outre : (a) une pluralité de réservoirs de stockage, contenant chacun des microbulles présentant une caractéristique de taille différente, ledit dispositif de commande étant conçu pour :
sélectionner l'un des réservoirs de stockage comportant en son sein les microbulles correspondant à la caractéristique des microbulles souhaitée ; et
provoquer l'administration des microbulles seulement depuis le réservoir de stockage sélectionné ; ou
(b) un dispositif de rayonnement destiné à transmettre une dose de rayonnement au tissu cible.

13. Système selon la revendication 1(i), ledit dispositif de commande étant en outre conçu pour distribuer les microbulles depuis le dispositif d'administration en fonction au moins en partie d'un profil d'administration prédéfini.

14. Système selon la revendication 13, ledit profil d'administration : (a) étant déterminé en fonction au moins en partie de la caractéristique des microbulles distribuées, éventuellement
ladite caractéristique des microbulles distribuées comprenant au moins l'une parmi un diamètre, une distribution de taille, une composition d'enveloppe, une composition de gaz ou une composition de noyau de liquide ; ou
(b) comprenant au moins un élément parmi une dose, une concentration, une vitesse d'administration, un moment d'administration ou une pression d'administration des microbulles.

15. Système selon la revendication 1(i), comprenant en outre : (a) un dispositif de préparation destiné à préparer les microbulles en fonction au moins en partie de la caractéristique souhaitée ; ou
(b) ledit dispositif de commande étant en outre conçu pour acquérir une caractéristique du tissu cible et déterminer la caractéristique souhaitée des microbulles en fonction au moins en partie de la caractéristique du tissu cible.

16. Système selon la revendication 1(ii) : (a) ledit manipulateur comprenant un filtre présentant une caractéristique de taille déterminée ;
(b) ledit transducteur étant conçu pour appliquer un champ acoustique présentant au moins une bande de fréquences de façon à détruire les microbulles présentant une taille en dehors d'une plage de tailles souhaitée ; ou
(c) ledit dispositif de commande étant en outre conçu pour acquérir une caractéristique du tissu cible et déterminer la caractéristique souhaitée des microbulles en fonction au moins en partie de la caractéristique du tissu cible.

17. Système selon la revendication 1(ii), ledit dispositif de commande étant en outre conçu pour administrer les microbulles en fonction d'un profil d'administration prédéfini.

18. Système selon la revendication 17, ledit profil d'administration : (a) étant déterminé en fonction au moins en partie d'une seconde caractéristique des microbulles, éventuellement
ladite seconde caractéristique des microbulles comprenant au moins une caractéristique parmi un diamètre, une distribution de taille, une composition d'enveloppe ou une composition de noyau de gaz et/ou de liquide ; ou
(b) comprenant au moins un élément parmi une dose, une concentration, une vitesse d'administration, un moment d'administration ou une pression d'administration des microbulles.

19. Système selon la revendication 1(ii), comprenant en outre un dispositif de préparation destiné à préparer les microbulles en fonction au moins en partie de la caractéristique souhaitée, sensiblement toutes les microbulles préparées présentant la caractéristique souhaitée.
